# EUROPEAN PATENT SPECIFICATION

(11) **EP 4 172 594 B1**
(45) Date of publication and mention of the grant of the patent: **15.04.2026**
(21) Application number: 21736615.2
(22) Date of filing: 29.06.2021
(51) Int. Cl.: G01N 21/23, G01N 33/18, G01N 15/06, G01N 21/21, G01J 4/04, G01N 21/53, G01N 15/00

(54) **A METHOD TO PRODUCE A MATCHED PAIR OF POLARIZING FILTERS AND A METHOD AND APPARATUS TO DETERMINE THE CONCENTRATION OF BIREFRINGENT PARTICLES USING A PAIR OF POLARIZING FILTERS**
EIN VERFAHREN ZUR HERSTELLUNG EINES ANGEPASSTEN POLARISIERENDEN FILTERPAARS UND EIN VERFAHREN UND EINE VORRICHTUNG ZUR BESTIMMUNG DER KONZENTRATION DOPPELBRECHENDER PARTIKEL MIT EINEM POLARISIERENDEN FILTERPAAR
PROCEDE DE PRODUCTION D'UNE PAIRE DE FILTRES POLARISANTS APPARIEE ET PROCEDE ET APPAREIL PERMETTANT DE DETERMINER LA CONCENTRATION DE PARTICULES BIREFRINGENT A L'AIDE D'UNE PAIRE DE FILTRES POLARISANTS

(30) Priority: 30.06.2020 EP 20183105
(43) Date of publication of application: 03.05.2023
(73) Proprietor: Universiteit Gent, 9000 Gent (BE); HIS MAJESTY THE KING IN RIGHT OF CANADA, AS REPRESENTED BY THE MINISTER OF NATIONAL DEFENCE, Ottawa, ON K1A 0K2 (CA)
(72) Inventor: NEUKERMANS, Griet, 9473 Welle (BE); FOURNIER, Georges, Québec, G3B 1N9 (CA)
(74) Representative: Ipsilon Belgium
(86) International application number: PCT/EP2021/067853
(87) International publication number: WO 2022/002939

(56) References cited:
- WO-A1-2017/099755
- US-A1- 2005 122 514
- LUC BEAUFORT ET AL: "Optical measurements to determine the thickness of calcite crystals and the mass of thin carbonate particles such as coccoliths", NATURE PROTOCOLS, vol. 9, no. 3, 20 February 2014 (2014-02-20), pages 633 - 642, XP055148654, ISSN: 1754-2189, DOI: 10.1038/nprot.2014.028
- ANONYMOUS: "The New DigitalMicroscope Series Leica DM for Life Sciences Simply Microscopy! Perfection At Work", 2004, Germany, pages 1 - 18, XP055759183, Retrieved from the Internet <URL:https://www.microscopy.uk.com/pdfs/Leica_DM4000B_45_50_60.pdf> [retrieved on 20201211]

## Description

### Field of the invention

The present invention relates to a method to produce a matched pair of polarizing filters. The matched pair of polarizing filters obtainable by such method allows to optimize the detection of depolarized light caused by a sample comprising birefringent particles. The matched pair of polarizing filters obtainable by such method preferably has an extinction ratio lower than 10⁻⁵. The invention also relates to a method to analyze a sample comprising birefringent particles suspended in a fluid, for example suspended in a liquid, using a matched pair of polarizing filters. In particular the invention relates to a method to determine the concentration of birefringent particles suspended in a fluid using a matched pair of polarizing filters. Furthermore, the invention relates to an apparatus for analyzing a sample comprising birefringent particles suspended in a fluid, for example suspended in a liquid, using a matched pair of polarizing filters. In particular the invention relates to an apparatus for determining the concentration of birefringent particles suspended in a fluid using a matched pair of polarizing filters.

### Background art

Nowadays, mitigation of climate changes induced by carbon dioxide (CO₂) emissions is a major challenge. Oceans take up an important part of these emissions. Up to now, the capacity to store carbon dioxide in the deep ocean is poorly known and underestimated. Carbon storage in the ocean is governed by two mechanisms : the solubility pump and the biological carbon pump. The biological carbon pump refers to a set of processes by which inorganic carbon (such as carbon dioxide) is fixed into organic material via photosynthesis and then sequestered away from the atmosphere generally by transport into the deep ocean. The biological carbon pump comprises two pumps: the organic carbon pump driven by photosynthesis fixation of CO₂ into particulate organic carbon (POC) by phytoplankton of which part gets exported to the deep ocean and the carbonate pump referring to the formation of calcium carbonate CaCO₃ (PIC) by calcifying organisms and its transport to the deep ocean.

Central to the understanding of the biological carbon pump is the accurate and precise measurement of the concentrations of PIC and POC. Up to now our understanding of PIC cycling has been limited because of inadequate sampling and analysis techniques to measure the concentration and downward flux of PIC and in particular the spatial and temporal variation of PIC in the oceans.

Previous attempts to measure the concentration of PIC are based on methods injecting an acid into a suspension and measuring the change in pH or through filtration of a known volume of seawater and subsequent laboratory determination of PIC or microscopy analysis. Such methods have a number of drawbacks. They require ship support, are labor-intensive, intrusive and do not allow autonomous in situ operation.

US 7,030,981 B2 describes a method and apparatus for measuring the concentration of CaCO₃ particles comprising a first linear polarizer and a second linear polarizer having polarization axes perpendicular to each other. The extreme sensitivity of the alignment of the polarizers of such apparatus is a serious drawback of such apparatus. Minute mechanical torsions induced for example by vibrations and changing water pressure with depth may cause too much random fluctuation in the signal that measurement of CaCO₃concentration is impossible. Luc Beaufort et al. published a document on "Optical measurements to determine the thickness of calcite crystals and the mass of thin carbonate particles such as coccoliths", NATURE PROTOCOLS, vol. 9, no. 3, 20 February 2014 (2014-02-20), pages 633-642, ISSN: 1754-2189, DOI: 10.1038/n prot.2014.028 which relies on an optical system comprising a polarizer and an analyzer (oriented at 90° from the polarizer).
WO 2017/099755 A1 describes a polarization microscope that uses spectral-encoding of the polarization state for cellular imaging, comprising an optically couples first polarizer, a first retarder, a second retarder and a second polarizer.
US 2005/122514 A1 describes a circular polarized light method and device for determining wall thickness and orientations of fibrils of cellulosic fibres, based on circular polarized light microscopy system.

### Summary of the invention

The present invention is directed to a method to produce a matched pair of polarizing filters as defined in appended independent claim 1, and to an apparatus for analyzing a sample comprising birefringent particles suspended in a fluid as defined in appended independent claim 9. Particular embodiments of the invention are defined in appended dependent claims 2-8 and 10-15. The scope of the invention is solely defined by the appended claims.

It is an object of the present invention to provide a method to manufacture a matched pair of polarizing filters having an extinction ratio lower than 10⁻⁵, lower than 5.10⁻⁶ or even lower than 1.10⁻⁶.

It is an object of the present invention to provide a method and an apparatus using a matched pair of polarizing filters to analyze a sample comprising birefringent particles.

It is an object of the present invention to provide a method and an apparatus using a matched pair of polarizing filters to determine the concentration of birefringent particles suspended in a fluid such as a liquid.

It is an object of the present invention to provide a method and apparatus to determine the concentration of calcium carbonate particles or the concentration of polymer particles, for example the concentration of calcium carbonate particles or the concentration of polymer particles suspended in a fluid, for example in water.

It is an object of the present invention to provide a method and an apparatus that allows to detect even small concentrations of birefringent particles suspended in a fluid such as a liquid and/or allows to detect small variations in the concentration of birefringent particles suspended in a fluid such as a liquid.

It is an object of the present invention to provide a method and an apparatus to determine PIC concentration in oceanic environments.

It is an object of the present invention to provide a method and apparatus that allow to determine the concentration of birefringent particles suspended in a fluid even when mechanical torsions and/or vibrations are induced and in case of changing (water) pressure.

It is an object of the present invention to provide a method and an apparatus to determine the concentration of polymer particles, for example in water such as seawater.

It is an object of the present invention to provide a method and an apparatus to determine the concentration of birefringent particles suspended in a fluid using circular polarizers whereby the method and apparatus are not wavelength sensitive.

It is another object of the present invention to provide a method and an apparatus to determine the concentration of birefringent particles suspended in a fluid whereby the signal that is recorded by the detector solely comes from light depolarized by the birefringent sample, for example by the birefringent particles suspended in the fluid.

It is still a further object of the present invention to provide a non-intrusive method to analyze a sample comprising birefringent particles either quantitatively or qualitatively.

Furthermore, it is an object to provide a method and an apparatus for analyzing a sample comprising birefringent particles in situ that functions autonomously, in particular to provide a method and apparatus for determining the concentration of birefringent particles suspended in a fluid, for example in water.

Additionally, it is an object of the present invention to provide a method and apparatus to optimize the detection of depolarized light caused by a sample comprising birefringent particles.

According to a first aspect of the present invention a method to produce a matched pair of polarizing filters comprising a first and second polarizing filter is provided. The first polarizing filter comprises a first linear polarizer and a first quarter-wave optical retarder (first phase plate) and the second polarizing filter comprises a second linear polarizer and a second quarter-wave optical retarder (second phase plate). The method allows to produce a matched pair of polarizing filters suitable to detect depolarized light caused by a sample comprising birefringent particles, for example depolarized light caused by a sample comprising birefringent particles suspended in a fluid. The method comprises the steps of
- providing a beam of light from a light source along a propagation axis, preferably providing a beam of parallel light;
- providing a first, a second, a third and a fourth rotation stage, preferably oriented perpendicular or substantially perpendicular to the propagation axis of the beam of light, preferably providing a first, a second, a third and a fourth high precision rotation stage oriented perpendicular or substantially perpendicular to the propagation axis of the beam of light;
- mounting a first linear polarizer having a first transmission axis in the first rotation stage with the first transmission axis in a particular position, referred to as the first position;
- mounting a second linear polarizer having a second transmission axis in the fourth rotation stage;
- rotating the second linear polarizer to obtain maximum extinction of the beam of light. Maximum extinction is obtained if the second transmission axis of the second linear polarizer is perpendicular to the first transmission axis of the first linear polarizer;
- inserting a first quarter-wave optical retarder having a first optical axis in the second rotation stage, the first optical axis of the first quarter-wave optical retarder is thereby preferably oriented perpendicular or substantially perpendicular to the propagation axis of the beam of light;
- rotating the first quarter-wave optical retarder to obtain maximum extinction of the beam of light;
- rotating the first optical axis of the first quarter-wave optical retarder over a first angle in a first direction, the first angle is preferably an angle of (about) 45 degrees, more preferably an angle of 45 degrees plus or minus 0.10 degrees, for example an angle of 45 degrees plus or minus 0.05;
- inserting a second quarter-wave optical retarder having a second optical axis in the third rotation stage, the second optical axis of the second quarter-wave optical retarder is thereby preferably oriented perpendicular or substantially perpendicular to the propagation axis of the beam of light;
- rotating the second optical axis of the second quarter-wave optical retarder in a second direction over a second angle to obtain maximum extinction of the beam of light, the second direction being opposite to the first direction of the rotation of the first optical axis of the first quarter-wave optical retarder as viewed from the light source. It is clear that the first angle and the second angle have opposite signs as viewed from the light source. When the maximum extinction is reached, the correct orientation of the second quarter-wave optical retarder will be reached. In this position the second angle is 45 degrees (plus or minus 0.05 degrees), although the method does not require to measure the second angle;
- securing the first linear polarizer and the first quarter-wave optical retarder together to form the first polarizing filter and securing the second linear polarizer and the second quarter-wave optical retarder together to form the second polarizing filter. By securing the first linear polarizer and the first quarter-wave optical retarder together, the relative positions of the first linear polarizer and the first quarter-wave optical retarder, in particular the relative position of the first transmission axis and the first optical axis are secured. Similarly, by securing the second linear polarizer and the second quarter-wave optical retarder, the relative positions of the second linear polarizer and second wave plate, in particular the relative position of the second transmission axis and second optical axis are secured. The first linear polarizer and the first quarter-wave optical retarder and similarly the second linear polarizer and the second quarter-wave optical retarder can be secured by any technique known in the art. A preferred technique to secure them comprises gluing.

The first, second, third and fourth rotation stage are positioned subsequently after each other, with the first rotation stage being closest to the light source and the fourth rotation stage being farthest from the light source.

A linear polarizer is a device that selectively allows the passage of only certain orientations of plane polarized light. At one orientation, it might allow the passage of only vertically polarized light, while if rotated by 90 degrees it would allow the passage of only horizontally polarized light. Preferably, a linear polarizer used according to the present invention has an extinction ratio lower than 10⁻⁵, lower than 5.10⁻⁶, lower than 3.10⁻⁶ or lower than 1.10⁻⁶. The extinction ratio of a linear polarizer is the ratio of the minimum transmission of the polarizer to the maximum transmission of the polarizer. The minimum transmission occurs when the transmission axis of the polarizer is perpendicular to the plane of polarization of an incident polarized beam; the maximum transmission occurs when the transmission axis of the polarizer is parallel to the plane of polarization of an incident polarized beam.

An optical retarder is a polarization device designed to produce a specified phase difference between exiting beams for two orthogonal incident polarization states. A quarter-wave optical retarder converts linearly polarized light in circularly polarized light or vice versa. The thickness of the optical retarder is preferably adjusted to give rise to a phase difference of a quarter of the wavelength λ (or wavelength range Δλ) it has been designed for. Preferably, a quarter-wave optical retarder used according to the present invention is of high quality. Preferably, a quarter-wave optical retarder used according to the present invention gives a retardance of λ/4± λ/350.

For the production of a matched pair of polarizing filters according to the present invention, a pair of quarter-wave optical retarders is selected such that the difference between the retardances of the first and second quarter-wave optical retarders does preferably not exceed λ/1000 and is more preferably lower than λ/10000, with λ being the design wavelength.

A linear polarizer has a transmission axis and a quarter-wave optical retarder has an optical axis. The transmission axis of a linear polarizer and/or the optical axis of a quarter-wave optical retarder are preferably oriented in a plane perpendicular or substantially perpendicular to the propagation axis of the beam of light from the light source.

Preferably, a linear polarizer has a planar structure defining a plane with the transmission axis of the linear polarizer embedded/oriented in the plane of the linear polarizer.

Preferably, a quarter-wave optical retarder of a polarizing filter has a planar structure defining a plane with the optical axis of the quarter-wave optical retarder embedded/oriented in the plane of the quarter-wave optical retarder.

The first polarizing filter comprises a first linear polarizer having a first transmission axis and a first quarter-wave optical retarder having a first optical axis and the second polarizing filter comprises a second linear polarizer having a second transmission axis and a second quarter-wave optical retarder having a second optical axis.

Preferably, each of the first transmission axis, the first optical axis, the second transmission axis and the second optical axis are oriented in a plane perpendicular or substantially perpendicular to the propagation axis of the beam of light from the light source.

Preferably, the first transmission axis is embedded in the plane of the first linear polarizer, the first optical axis is embedded in the plane of the first quarter-wave optical retarder, the second transmission axis is embedded in the plane of the second linear polarizer and the second optical axis is embedded in the plane of the second quarter-wave optical retarder.

The first optical axis and the first transmission axis define a first angle (a first included angle) and the second optical axis and the second transmission axis define a second angle (a second included angle). Preferably, the first angle and the second angle are 45 degrees (preferably 45 degrees plus or minus 0.05 degrees), with the first angle and the second angle having opposite signs as viewed from the light source.
The first angle is for example +45 degrees (preferably +45 degrees plus or minus 0.05 degrees), whereas the second angle is -45 degrees (preferably + 45 degrees plus or minus 0.05 degrees). In alternative embodiments, the first angle is -45 degrees (preferably -45 degrees plus or minus 0.05 degrees), whereas the second angle is +45 degrees (preferably +45 degrees plus or minus 0.05 degrees).

Preferably, the first polarizing filter of the matched pair of polarizing filters is polarizing incident light into circularly polarized light of a first handedness sense and the second polarizing filter of the matched pair of polarizing filters is polarizing incident light into circularly polarized light of a second handedness sense, whereby the first handedness sense and the second handedness sense are opposite as viewed from the light source.

In a first example the first polarizing filter is configurable to polarize incident light into left circularly polarized light, while the second polarizing filter is configurable to polarize incident light into right circularly polarized light.
In an alternative example the first polarizing filter is configurable to polarize incident light into right circularly polarized light, while the second polarizing filter is configurable to polarize incident light into left circularly polarized light.

As light source, any type of light source known in the art can be considered. The light source may comprise an unpolarized or polarized light source. In case a polarized light source is used, it is clear that the polarization direction of the light source and the first linear polarizer are preferably aligned. The light source comprises preferably a pulsed light source. Preferred light sources comprise (pulsed) light sources within a wavelength range where absorption by the fluid itself and/or by particles and other substances present in the sample are minimal, for example pulsed light sources within a wavelength range where absorption by particles and other substances present in the fluid such as water or seawater are minimal. The beam of light from the light source has preferably a central wavelength in the wavelength range between 530 and 650 nm or in the wavelength range between 590 nm and 650 nm. The beam of light does not require a very narrow selection of wavelength, although an emission wavelength band with a full width at half maximum (FWHM) equal to or less than 50 nm or more preferably equal to or less than 20 nm.

Preferably, the light source has an emission wavelength band having a central wavelength between 530 nm and 650 nm, for example between 590 nm and 650 nm, and a full width at half maximum of 50 mm, for example a full width at half maximum of 20 mm. A particular preferred example of a light source has a central wavelength of 650 nm and a full width at half maximum of 20 nm.

A preferred light source comprises a LED (Light Emitting Diode) light source, for example a pulsed LED source, for example a pulsed LED source having a full width at half maximum of 20 nm and a center wavelength of 650 nm.

As detector, any type of detector suitable to measure light from the light source, preferably any type of detector suitable to measure a pulsed light from a light source can be considered.

Preferred detectors comprise silicon photodetectors, such as amplified, switchable gain, silicon photodiode detectors.

The method to produce a matched pair of polarizing filters according to the present invention allows to obtain a matched pair of polarizing filters having an extinction ratio lower than 10⁻⁵. In particular embodiments of the present invention a matched pair of polarizing filters having an extinction ratio lower than 5.10⁻⁶, lower than 3.10⁻⁶ or even lower than 1.10⁻⁶ is obtained.
The extinction ratio of a matched pair of polarizing filters is defined as the ratio of the intensity of light transmitted through the matched pair of polarizing filters to the intensity of unpolarized light (or polarized light) impinging on the first polarizer.

Although the extinction ratio of circular polarizing filters is generally wavelength sensitive and thus not usable with a light source having a wavelength band, for example a wavelength band of 20 nm, such a light emitting diode, the method to produce a matched pair of polarizing filters according to the present invention allows to obtain a matched pair of polarizing filters that is wavelength insensitive because of the geometric arrangement of the polarizing filters. The matched pair of polarizing filters reaches the low extinction ratio preferably at least in the wavelength range of the emission wavelength band defined by the full width at half maximum (FWHM) of the beam of light of the light source.

Accordingly, a matched pair of polarizing filters obtainable by the method to produce a matched pair of polarizing filters according to the present invention can use light emitting diodes as light source. Furthermore, a method to analyze a sample using such matched pair of polarizing filters and/or an apparatus comprising such matched pair of polarizing filters can use light emitting diodes as light source.

As described above the first quarter-wave optical retarder is rotated in a first direction over a first angle, more particularly an angle of 45 degrees. Preferably, the accuracy of the first angle is plus or minus 0.1 degrees and more preferably plus or minus 0.05 degrees. The second quarter-wave optical retarder is rotated in a second direction, opposite to the first direction, over a second angle to obtain maximum extinction of the beam of light. When the maximum extinction is reached, the correct orientation of the of the second quarter-wave optical retarder is reached. Although the method does not require to measure the second angle, the second angle is supposed to be 45 degrees. The accuracy of the second angle is preferably plus or minus 0.1 degrees and more preferably plus or minus 0.05 degrees.

The method to produce a matched pair of polarizing filters has the advantage to be self-correcting. A small deviation of the first angle will be corrected by the rotation of the second quarter-wave optical retarder to obtain maximum extinction.

Preferably the difference in retardance between the first quarter-wave optical retarder and the second quarter-wave optical retarder is lower than 10⁻³ for the wavelength (or wavelength range) it is designed for. More preferably, the difference in retardance between the first quarter-wave optical retarder and the second quarter-wave optical retarder is lower than 10⁻⁴ for the wavelength (or the wavelength range) it is designed for.

According to a second aspect of the present invention a method to analyze a sample comprising birefringent particles suspended in a fluid, for example in a liquid such as water or seawater, as defined in appended claim 4 is provided. The method is in particular suitable to determine the concentration of birefringent particles suspended in a fluid, for example in a liquid such as water or seawater.

The extinction ratio of a matched pair of polarizing filters is defined as the ratio of the intensity of light transmitted through the matched pair of polarizing filters to the intensity of unpolarized light (or polarized light) impinging on the first polarizer.

The matched pair of polarizing filters is obtained by the above described method (a method as defined in appended claim 1 or claim 2) to produce a matched pair of polarizing filters. As first linear polarizer, second linear polarizer, first quarter-wave optical retarder and second quarter-wave optical retarder, the first linear polarizer, the second linear polarizer, the first quarter-wave optical retarder and the second quarter-wave optical retarder as described above are used, in the configuration obtained by the method (a method as defined in appended claim 1 or claim 2) to produce a matched pair of polarizing filters as described above.

As light source, any type of light source known in the art can be considered. The light source may comprise an unpolarized or polarized light source. In case a polarized light source is used, it is clear that the polarization direction of the light source and the first linear polarizer are preferably aligned. The light source comprises preferably a pulsed light source. Preferred light sources comprise (pulsed) light sources within a wavelength range where absorption by the fluid itself and/or by particles and other substances present in the sample are minimal, for example pulsed light sources within a wavelength range where absorption by particles and other substances present in the fluid such as water or seawater are minimal. The beam of light from the light source has preferably a central wavelength in the wavelength range between 530 and 650 nm or in the wavelength range between 590 nm and 650 nm. The beam of light does not require a very narrow selection of wavelength, although an emission wavelength band with a full width at half maximum (FWHM) equal to or less than 50 nm or more preferably equal to or less than less than 20 nm.

Preferably, the light source has an emission wavelength band having a central wavelength between 530 nm and 650 nm, for example between 590 nm and 650 nm, and a full width at half maximum of 50 nm, for example a full width at half maximum of 20 mm. A particular preferred example of a light source has a central wavelength of 650 nm and a full width at half maximum of 20 nm.

A preferred light source comprises a LED (Light Emitting Diode) light source, for example a pulsed LED source, for example a pulsed LED source having a full width at half maximum of 20 nm and a center wavelength of 650 nm.

As detector, any type of detector suitable to measure light from the light source, preferably any type of detector suitable to measure a pulsed light from a light source can be considered.

Preferred detectors comprise silicon photodetectors, such as amplified, switchable gain, silicon photodiode detectors.

The third beam of light corresponds with the depolarized light caused by the sample, more particularly caused by the birefringent particles suspended in the fluid, for example in the liquid. Accordingly, in case the detector detects a third beam, this means that the sample analyzed is a birefringent sample, for example a sample comprising birefringent particles suspended in a fluid, for example in a liquid such as water or seawater.
In particular embodiments of the invention, the third beam detected by the detector allows to determine the concentration of the birefringent particles suspended in fluid, for example in a liquid such as water of seawater.

Because of the configuration of the matched pair of polarizing filters and/or because of the low extinction ratio of the matched pair of polarizing filters, the method to analyze a birefringent sample, for example a sample comprising birefringent particles suspended in a fluid such as a liquid, allows to determine the concentration of birefringent particles even if the concentration of the birefringent particles suspended in the fluid is low, for example lower than 50 micromol/L, lower than 10 micromol/I, lower than 1 micromol/L, lower than 0.1 micromol/L, lower than 0.01 micromol/L, for example 0.005 micromol/L.

Birefringent particles comprise for example calcium carbonate, quartz, celestite, barite, kaolinite, chlorite, illite, vermiculite, orthoclase, plagioclase, montmorillonite, plastic (also referred to as microplastic) or combinations thereof.

The particles have preferably a size ranging between 1 µm and 5 mm, for example a size ranging between 2 µm and 3 mm or between 2 µm and 1 mm, such as 10 µm, 100 µm, 200 µm or 500 µm.

The method is suitable to analyze the concentration of calcium carbonate in water, for example in seawater.

Furthermore, the method is suitable to analyze the presence or concentration of polymer particles in water.

According to a third aspect of the present invention an apparatus for analyzing a sample comprising birefringent particles, is provided. The apparatus comprises a light source for emitting a beam of light along a propagation axis, a matched pair of polarizing filters produced by the method described above (defined in claim 1 or claim 2), said matched pair of polarizing filters comprising a first polarizing filter and a second polarizing filter and a detector. The light source, the matched pair of polarizing filters and the detector are arranged in such a way that the beam of light emitted by the light source subsequently can pass through the first polarizing filter, can impinges on the sample to be analyzed, can pass through the second polarizing filter before being detected by the detector. The first polarizing filter is preferably positioned close to the light source, whereas the second polarizing filter is preferably positioned close to the detector. The first polarizing filter comprises a first linear polarizer and a first quarter-wave optical retarder and is configurable to polarize incident light into circularly polarized light of a first handedness sense viewed form the light source. The second polarizing filter comprises a second linear polarizer and a second quarter-wave optical retarder and is configurable to polarize incident light into circularly polarized light of a second handedness sense, whereby the first handedness sense and the second handedness sense are opposite as viewed from the light source. The matched pair of polarizing filters has preferably an extinction ratio lower than 10⁻⁵. More preferably, the matched pair of polarizing filters has an extinction ratio lower than 5.10⁻⁶, lower than 3.10⁻⁶ or even lower than 1.10⁻⁶.

The matched pair of polarizing filters is obtained by the above described method to produce a matched pair of polarizing filters. As first linear polarizer, second linear polarizer, first quarter-wave optical retarder and second quarter-wave optical retarder, the first linear polarizer, the second linear polarizer, the first quarter-wave optical retarder and the second quarter-wave optical retarder as described above are preferably used, preferably in the configuration obtained by the method to produce a matched pair of polarizing filters as described above.

As light source, any type of light source known in the art can be considered. The light source may comprise an unpolarized or polarized light source. In case a polarized light source is used, it is clear that the polarization direction of the light source and the first linear polarizer are preferably aligned. The light source comprises preferably a pulsed light source. Preferred light sources comprise (pulsed) light sources within a wavelength range where absorption by particles and other substances present in the sample are minimal, for example pulsed light sources within a wavelength range where absorption by particles and other substances present in the fluid such as water or seawater are minimal. The beam of light from the light source has preferably a central wavelength in the wavelength range between 530 and 650 nm or in the wavelength range between 590 nm and 650 nm. The beam of light does not require a very narrow selection of wavelength, although an emission wavelength band with a full width at half maximum (FWHM) equal to or less than 50 nm or more preferably equal or less than 20 nm.

Preferably, the light source has an emission wavelength band having a central wavelength between 530 nm and 650 nm, for example between 590 nm and 650 nm, and a full width at half maximum of 50 mm, for example a full width at half maximum of 20 mm. A particular preferred example of a light source has a central wavelength of 650 nm and a full width at half maximum of 20 nm.

A preferred light source comprises a LED (Light Emitting Diode) light source, for example a pulsed LED source, for example a pulsed LED source having a full width at half maximum of 20 nm and a center wavelength of 650 nm.

As detector, any type of detector suitable to measure light from the light source, preferably any type of detector suitable to measure a pulsed light from a light source can be considered.

Preferred detectors comprise silicon photodetectors, such as amplified, switchable gain, silicon photodiode detectors.

An apparatus according to the present invention allows to measure the light depolarized by a sample comprising birefringent particles. Consequently, the apparatus is suitable to analyze, either qualitatively or quantitatively, a sample comprising birefringent particles.

In particular an apparatus according to the present invention allows to measure the depolarization fraction, i.e. the fraction of circularly polarized light transmitted by the first polarizing filter that gets depolarized by birefringent particles suspended in a fluid (for example water) and then passes through the second polarizing filter and impinges on the detector.

In preferred embodiments, the apparatus according to the present invention comprises a transmissometer. A transmissometer is defined as an instrument for measuring the transmission of light through a fluid (such as the atmosphere or water). The analyzed sample has typically a length (path length) of at least 5 cm, for example a length of 7 cm, 10 cm or 15 cm. The volume of the analyzed sample corresponds with the path length x π x (beam diameter/2)² is typically at least 3 mL, for example at least 12 mL. The beam diameter is for example 8.5 mm.

In principle, the apparatus according to the present invention, for example the transmissometer according to the present invention, allows to analyze any type of birefringent sample. The apparatus according to the present invention is in particular suitable to analyze samples comprising birefringent particles, for example birefringent particles suspended in a fluid such as a gas or a liquid. Particularly preferred birefringent samples comprise samples having birefringent particles suspended in a liquid, for example birefringent particles suspended in water such as seawater.

For a sample comprising birefringent particles suspended in a fluid, for example in a liquid, the apparatus according to the present inventions allows to determine the concentration of birefringent particles in the fluid, for example in the liquid such as water or seawater.

The apparatus according to the present invention is suitable to determine PIC concentrations in seawater. PIC comprises biogenic particles and includes both calcite and aragonite polymorphs of calcium carbonate (CaCO₃) having extreme birefringent properties. The apparatus according to the present invention allow to determine PIC concentration in the global ocean environment, including oligotrophic and deep waters.

Furthermore, the apparatus according to the present invention is suitable to analyze the presence or concentration of polymer particles in a liquid, for example in water.

Because of the configuration of the matched pair of polarizing filters and/or because of the low extinction ratio of the matched pair of polarizing filters, the apparatus to analyze a birefringent sample, for example a sample comprising birefringent particles suspended in a fluid such as a liquid, allows to determine the concentration of birefringent particles even if the concentration of the birefringent particles suspended in the fluid is low, for example lower than 100 micromol/L, lower than 50 micromol/L, lower than 10 micromol/I, lower than 1 micromol/L, lower than 0.1 micromol/L, lower than 0.01 micromol/L, for example 0.005 micromol/L.

The first polarizing filter and/or the second polarizing filter of an apparatus according to the present invention are preferably rotatable and/or removable. Preferably, the first polarizing filter and/or the second polarizing filter are removable.

In preferred embodiments, the second polarizing filter, i.e. the filter in front of the detector of an apparatus is removable so that the apparatus has the additional advantage that the polarized and unpolarized transmission can be measured by the same apparatus. In such an embodiment, a gain switcher can be used to change the gain of the detector according to whether polarized or unpolarized transmission is recorded. Alternatively, a neutral density filter can be inserted in place of the removed polarizer to lower the unpolarized signal to the same range of amplitude as the polarized signal.

Optionally, the apparatus according to the present invention comprises one or more additional component such
- a beam splitter, for example a polarized beam splitter; and/or
- one or more baffles, for example to protect the birefringent sample or a zone comprising the birefringent sample from incident light, for example from direct sun and/or skylight, and/or from background light of diffused underwater light. In preferred embodiments the apparatus comprises one or more baffle protecting the zone comprising the birefringent sample and the windows bounding said zone) from incident light; and/or
- one or more pressure windows, preferably one or more pressure window comprising a material that depolarizes as little as possible under a pressure stress. A preferred pressure window comprises an amorphous SiO₂ or a coated Schott Glass SF57 pressure window; and/or
- one or more spectral filters, for example for blocking background incident light while letting through the beam of light, for example collimated beam of the light source; and/or
- one or more lenses, for example one or more collimating lens; and/or
- one or more precision pinholes.

During use, in particular during use in water bodies exposed to sunlight, an apparatus according to the present invention is preferably oriented in such a way that the propagation axis of the beam of light from the light source is oriented vertically with the light source emitting the beam of light upward. The detector is preferably oriented to detect the upwardly emitting beam of light.

An apparatus according to the present invention has a number of advantages compared to apparatuses known in the art.

A first advantage is its low sensitivity to mechanical torsions. The sensitivity to mechanical torsions of the polarizing filters of an apparatus according to the present invention is over an order of magnitude lower, preferably at least two orders of magnitude lower than the sensitivity to mechanical torsions of linear polarizers as for example described in US 7,030,981 B2.

A second benefit of an apparatus according to the present invention is that the circular depolarization signal from the birefringent sample for example from the birefringent particles is twice as large as the linear depolarization signal.

Although the maximum extinction ratio of circular polarizers quoted by commercial manufacturers is 2.0 x 10⁻³, which would not allow the detection of light depolarization due to birefringent particles in most oceanic environments, the apparatus according to the present invention has an extinction ratio of 1.0 x 10⁻⁵, 5.0 x 10⁻⁶, 3 x 10⁻⁶ or 1.0 x 10⁶. Consequently, the detection limit of an apparatus according to the present invention is increased by at least two orders of magnitude compared to devices known in the art.

Although the extinction ratio of circular polarization filters is generally wavelength sensitive and thus not usable with light emitting diodes as light source, the apparatus according to the present invention is wavelength insensitive because of the geometric arrangement of the polarizing filters. Consequently, the apparatus according to the present invention can use light emitting diodes as light source.

The apparatus according to the present invention can be used as sensor, for example as sensor to detect the presence, amount or concentration of a birefringent material, for example the presence, amount or concentration of birefringent particles in a fluid, for example in water or seawater. A preferred use of the apparatus according to the present invention is as calcium carbonate sensor. Another preferred use of the apparatus according to the present invention as a sensor to detect the presence and/or to determine the concentration of plastics, for example microplastics in a fluid such as water.

The apparatus according to the present invention functions as transmissometer. The transmissometer can be used to measure the transmission as well as the depolarization of light.

### Brief description of the drawings

The present invention will be discussed in more detail below, with reference to the attached drawings, in which:
- Figure 1 is a schematic illustration of a setup for the fabrication of a matched pair of polarizing filters;
- Figure 2 is a schematic illustration of the configuration of an apparatus for analyzing a birefringent sample according to an embodiment of the present invention:
- Figure 3 shows the extinction ratio in function of the second angle for two different matched pairs of polarizing filters according to an embodiment of the present invention.

### Description of embodiments

The present invention will be described with respect to particular embodiments and with reference to certain drawings but the invention is not limited thereto but only by the claims. The drawings are only schematic and are non-limiting. The size of some of the elements in the drawing may be exaggerated and not drawn on scale for illustrative purposes. The dimensions and the relative dimensions do not correspond to actual reductions to practice of the invention.

When referring to the endpoints of a range, the endpoints values of the range are included.

When describing the invention, the terms used are construed in accordance with the following definitions, unless indicated otherwise.

The term 'and/or' when listing two or more items, means that any one of the listed items can by employed by itself or that any combination of two or more of the listed items can be employed.

The terms first, second and the like in the description and in the claims, are used for distinguishing between similar elements and not necessarily for describing a sequence, either temporally, spatially, in ranking or in any other manner. It is to be understood that the terms so used are interchangeable under appropriate circumstances and that the embodiments of the invention described herein are capable of operation in other sequences than described or illustrated herein.

The term 'birefringence' refers to the optical property of a material to split a beam of light into two beams of unequal velocities (corresponding to two different refractive indices of the crystal) which subsequently recombine to form a beam of light that is no longer linearly polarized.

The term 'particle' refers to any type of small fragments of a material independent of the shape of such fragments. The term particle refers to single particles or to a plurality of particles.

The term 'analyzing' or 'analysis' refers to any qualitative and/or quantitative measurement or analysis, for example measuring the presence or absence of birefringent material and/or measuring the amount or concentration of birefringent material. In particular the term analyzing refers to measuring the presence or absence of birefringent particles, and/or determining the concentration of birefringent particles.

In preferred embodiments of the present invention the term 'analyzing' or 'analysis' refers to determining the concentration of particles suspended in a fluid, for example determining the concentration of particles suspended in a liquid.

The term 'fluid' refers to a medium such as a gas or a liquid. Preferred liquids comprise water such as sea water.

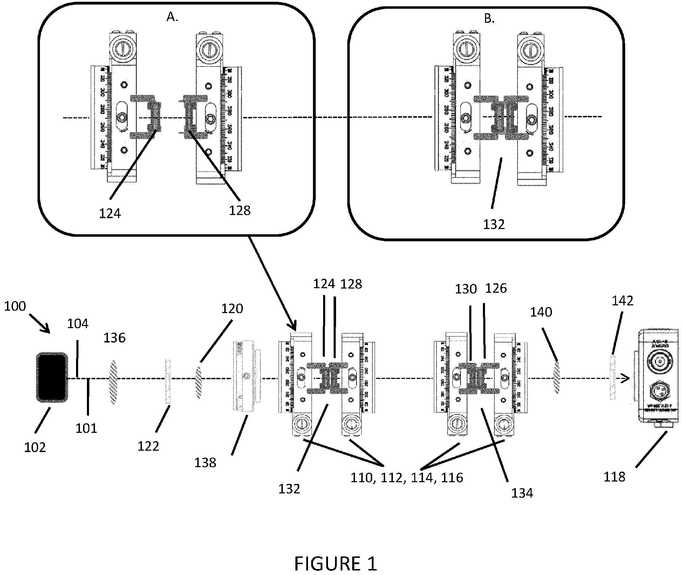

The method to optimize the setup 100 in order to obtain an optimal detection of depolarized light caused by a birefringent sample through the fabrication of a matched pair of polarizing filters having a low extinction ratio (high-rejection ratio) is illustrated in Figure 1.

First a beam of light 104 is generated from a light source 102. The beam of light 104 is oriented along an axis 101, referred to as the propagation axis of the beam of light 104. A preferred light source 102 to generate the beam of light 104 comprises a LED source. The beam of light 104 is preferably centered about a narrow spectral band, for example a spectral band equal or less than 20 nm full width at half height. The beam of light 104 has for example a spectral band equal to or less than 20 nm full width at half height with center wavelength 645 nm.

The beam of light 104 is preferably a parallel or nearly parallel beam of light, for example obtained using two lenses 136, 120 and a pinhole 122.

The setup 100 comprises four high precision rotation stages, respectively a first, a second, a third and a fourth high precision rotation stage 110, 112, 114 and 116. The high precision rotation stages 110, 112, 114 and 116 are oriented perpendicular or substantially perpendicular to the propagation axis 104 of the light beam 104. The setup 100 further comprises a detector 118. The detector 118 comprises for example a silicon photodiode detector. Preferably, a focusing lens (collimating lens) 140 and/or a precision pinhole 142 is/are provided between the fourth high precision rotation stage 116 and the detector 118.

A first linear polarizer 124 and a second linear polarizer 126 are mounted respectively in the first high precision rotation stage 110 and the fourth high precision rotation stage 116. The angle of the transmission axis of the first linear polarizer 124 is noted. The fourth rotation stage 116 is subsequently rotated until maximum extinction (minimum transmission) is obtained. At that point, the transmission axis of the first linear polarizer 124 and the transmission axis of the second linear polarizer 126 are at an angle of 90 degrees with respect to one another.

Subsequently, a first quarter-wave optical retarder (first phase plate) 128 is inserted in the second high precision rotation stage 112. The second high precision rotation stage 112 is rotated to obtain maximum extinction (minimum transmission). At that point, the optical axis of the first quarter-wave optical retarder 128 is precisely parallel to the polarization axis of the first linear polarizer 124. Subsequently, the optical axis of the first quarter-wave optical retarder 128 is rotated over 45 degrees as viewed from the light source 102. The accuracy of this rotation depends on the mechanical precision of the high precision rotation stage and can be easily less than a few milliradians.

**Once** the first quarter-wave optical retarder 128 has been set at 45 degrees, a second quarter-wave optical retarder (second phase plate) 130 is mounted in the third high precision rotation stage 114, just in front of the second linear polarizer 126. The second quarter-wave optical retarder 130 is rotated in the opposite sense as the first quarter-wave optical retarder 128 until once again maximum extinction (minimum transmission) is obtained. At this point, the optical axis of the second quarter-wave optical retarder 130 is at an angle of -45 degrees with the transmission axis of the linear polarizer 126 as viewed from the light source 102.

**At** that point a setup comprising a matched pair of circular polarizing filters, 132 and 134 with maximum mutual rejection ratio is obtained. The linear polarizer and the quarter-wave optical retarder for each pair of filter 132, 134 held in the rotation stages are then secured together, for example glued together. Preferably, the axes and side of the linear polarizers are marked to allow each filter assembly to be realigned when finally mounted, for example in a transmissometer.

Fig. 1A shows the preliminary setup of the first linear polarizer 124 and the first quarter-wave optical retarder 128. Fig. 1B shows the final setup of the first polarizer 124 and the first quarter-wave optical retarder 128 after assembling and securing (for example gluing).

Since the light acceptance angle of the transmissometer is about at least two degrees the angular dependence of the polarizing filters 132 and 134 should preferably be low. This can be achieved by using true zero order waveplates, i.e. waveplates comprising a single layer of polarizing material (either polymer material or an uniaxial crystal) bonded to an amorphous substrate. Such a true zero order waveplate has the lowest achievable retardance variation angle.

**The** setup 100 may further comprise a graduated iris 138 positioned before the first polarizing filter 132. Furthermore the setup 100 may comprise a lens 136 (imaging lens) between the light source 102 and the precision pinhole 122 and/or a lens 140 (a focusing lens) positioned between the fourth high precision rotation stage 116 and/or a precision pinhole 142 positioned between the fourth high precision rotation stage 116 and the detector 118, preferably between the lens 140 and the detector 118.

**The** assembly process of the polarizing filters 132, 134 as described above has the advantage to be self-correcting.

**The** best rejection ratio (extinction ratio) that can be achieved with the proposed process is determined by the maximum rejection ratio of the crossed linear polarizers and by the angular accuracy of the rotation stages used in the fabrication process. Preferably, one or more of the components of the setup are provided with an antireflection coating to minimize any potential mutual interaction. Most preferably, all components of the setup are provided with an antireflection coating.

### Reference signs Figure 1

- 100: setup
- 101: propagation axis of light beam
- 102: light source
- 104: beam of light
- 110: first high precision rotation stage
- 112: second high precision rotation stage
- 114: third high precision rotation stage
- 116: fourth high precision rotation stage
- 118: detector
- 120: focusing lens
- 122: precision pinhole
- 124: first linear polarizer
- 126: second linear polarizer
- 128: first quarter-wave optical retarder
- 130: second quarter-wave optical retarder
- 132: first polarizing filter
- 134: second polarizing filter
- 136: imaging lens
- 138: graduated iris
- 140: focusing lens
- 142: precision pinhole

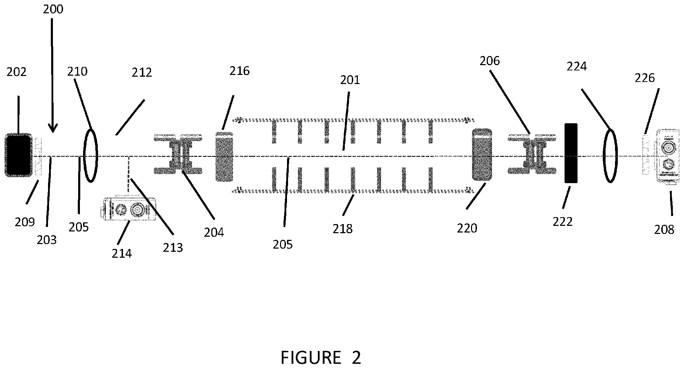

Figure 2 shows a schematic illustration of an apparatus 200 according to an embodiment of the present invention. The apparatus 200 is suitable to measure the depolarization of circularly polarized light by a birefringent sample 201, for example of a sample comprising birefringent particles suspended in water. The apparatus 200 is in particular suitable as transmissometer. The apparatus (transmissometer) measures the depolarization fraction, i.e. the fraction of circularly polarized light transmitted by the first polarizing filter that gets depolarized by birefringent particles suspended in a fluid (for example water) in the sample section of the apparatus and thus passes through the second polarizing filter and then impinges onto the detector. The apparatus has for example a detection limit for the depolarization fraction lower than 3.10⁻⁶ m⁻¹, which is roughly equivalent to 0.005 mmol CaCO₃m⁻³ for a pathlength of 15 cm.

The apparatus 200 comprises an emitting section, a sample section and a receiver section. The emitting section comprises a light source 202 and a first polarizing filter (first circular polarizer) 204 of a first handedness. The sample section comprises a sample holder, for example a column for receiving and/or holding a sample, for example water comprising birefringent particles. The receiver section comprises a second polarizing filter (second circular polarizer) 206 having a second handedness, opposite from the first handedness as seen from the light source and comprises a detector 208.

Each of the first and the second polarizing filters 204, 206 comprise a linear polarizer and a quarter-wave optical retarder. The first polarizing 204 filter comprises a first linear polarizer having a first transmission axis and a first quarter-wave optical retarder having a first optical axis. The second polarizing filter 206 comprises a second linear polarizer having a second transmission axis and a second quarter-wave optical retarder having a second optical axis. The first linear polarizer, the first quarter-wave optical retarder, the second linear polarizer and the second quarter-wave optical retarder are preferably oriented with their plane perpendicular to the propagation axis of the beam of light. The first transmission axis and the second transmission axis are preferably oriented perpendicular to each other. The (included) angle defined by the first optical axis and the first transmission axis and the (included) angle defined by the second optical axis and the second transmission axis are preferably equal or substantially equal and most preferably equal to 45 degrees. The angle between the first optical axis and the first transmission axis and the angle between the second optical axis and the second transmission axis have preferably opposite signs as viewed from the light source. The angle between the first optical axis and the first transmission axis is for example +45 degrees whereas the angle between the second optical axis and the second transmission axis is -45 degrees.

**A** beam of light 203 emitting from the light source 202 subsequently passes through the first polarizing filter 204, impinges on the sample 201 and passes through the second polarizing filter 206 before being detected by a detector 208.

The light source 202 comprises for example a LED source generating a beam 203 of light pulses along propagation axis 205. The beam of light 202 is preferably centered about a narrow spectral band, preferably a spectral band equal to or less than 20 nm full width at half amplitude with center wavelength 645 nm. The beam of light 203 passes preferably through a pinhole 209.

Preferably, the beam of light 203 is collimated in a parallel or nearly parallel beam by a collimating lens 210.

The parallelism of the beam is set by the ratio of diameter of the pinhole 209 to the focal length of the collimating lens 210.

It can be preferred that part of the light is then picked off by a beam splitter 212. The part 213 diverted by the beam splitter 212 may illuminate a reference detector 214 used as a monitor of the intensity of the light source 202.

Contrary to transmissometers known in the art, the transmissometer according to an embodiment of the present invention has preferably a polarized beam splitter 212 which diverts the linear polarization in the opposite orientation to the one of the first polarizing filter 204. This ensures that the maximum available light is sent through the first polarizing filter 204 and into the water column. Since LED's are unpolarized, the light diverted by the beam splitter 212 allows an accurate monitoring of the light transmitted through the first polarizing filter 204.

The apparatus 200 preferably comprises one or more pressure windows 216, 220. The material of the pressure window is preferably carefully chosen so that it has a minimum amount of stress-induced depolarization. Stress relieved amorphous SiO₂ (amorphous quartz) of coated SF57 glass are suitable materials for this purpose.

The unpolarized background light coming from the sun and sky light that penetrates through the water surface is preferably reduced to a level that will allow the detector 208 to operate. Therefore the apparatus 200 is preferably used in a vertical configuration with the light source emitting light in the upward direction and the detector facing in the downward direction. In this orientation the background light that enters the detector 208 comes from the sun and sky light illuminating the emitter window and supporting structure that are within the field of view of the detector and that are diffusely reflected by the window and supporting structures. For this reason it is preferred that any metal component of the emitter section that is in the field of view is black, for example black anodized.

Even with these precautions, it is preferred to further shield the area of the emitter section that is visible by the detector from direct sun and skylight. This can be achieved by the judicious use and positioning of light baffles 218 that shadow the sensitive area. These baffles 218 are preferably kept small and thin enough so as to not impede the free flow of water laterally through the water section. It is important to note that there is the limiting cone of light at which any radiation coming from above is transmitted through the water surface. That cone defines the limit of the underwater sky image that can illuminate the emitter surface. This limiting angle is 50 degrees. Water waves can distort the cone extend the illumination to an angle of 60 degrees. The disposition of a minimum number of the baffles preferably ensures that no part of this down welling light reaches the visible surface of the emitter. Direct shielding from the sun and sky can be achieved with a small number of baffles disposed around the emitter and receiver sections. There might however be another source of un-polarized light in the water column that comes from the upwelling light backscattered by underwater particles. Shielding from this source requires a larger set of baffles spaced along the entire length of the instruments open water column. Note that even in this case it is possible to achieve free lateral flow through the measurement column.

Preferably, the first polarizing filter 204 is the last element before the pressure window 216. In this way it is ensured that no depolarization of the circularly polarized light resulting from the first polarizing filter 204 occurs elsewhere than in the water sample.

The receiver section comprises preferably a pressure window 220, for example a stressrelieved amorphous quartz window followed immediately by a second polarizing filter 206 of the opposite handedness compared to the first polarizing filter 204 used in the emitter section. The axes of the linear polarization subcomponents of these circular filters must be carefully oriented perpendicular to one another in order to ensure that their rejection ratio is maintained over the wavelength range of the light source.

Preferably, the apparatus comprises a narrow spectral bandwidth optical filter 222 immediately positioned after the second polarizing filter 206. By introducing a spectral bandwidth optical filter 222 any background light coming through the detector 208 is reduced.

Preferably, the out of band optical density of the filter 222 is 10⁻⁴ (OD-4) or better over a wavelength range from 200 nm to 1200 nm or more to ensure a maximum amount of background light rejection. Preferably, the receiver section further comprises a lens (a collimating lens) 224 and/or a precision pinhole 226 . The lens 224 is preferably positioned after the second polarizing filter 206 and after the narrow band spectral filter 222.

**The** water column depolarized signal collection angle is set by the ratio of the diameter of the pinhole 226 and the focal length of the lens 220.

### Reference signs Figure 2

- 200: apparatus
- 201: sample
- 202: light source
- 203: beam of light
- 204: first polarizing filter
- 205: propagation axis of beam of light
- 206: second polarizing filter
- 208: detector
- 209: precision pinhole
- 210: collimating lens
- 212: polarized beam splitter
- 214: reference signal detector
- 216: pressure window
- 218: baffles
- 220: pressure window
- 222: narrow band spectral filter
- 224: collimating lens
- 226: precision pinhole

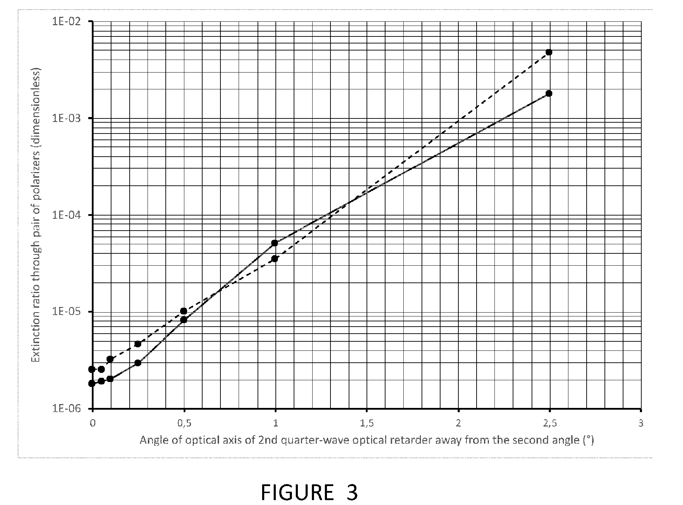

Figure 3 shows the extinction ratio that is obtained using two different matched pairs of polarizing filters, each built according to the fabrication method of the present invention. The obtained extinction is plotted in function of the second angle (i.e. the angle of the optical axis of the second quarter-wave optical retarder and the transmission axis of the second linear polarizer), in particular in function of the deviation of the second angle from 45 degrees (plus or minus 0.05 degrees).

Figure 3 illustrates that the intended extinction ratio is reached with a matched pair of polarizing filters according to the present invention. The first matched pair of polarizing filters reached an extinction ratio of 1.84 10⁻⁶, while the other matched pair of polarizing filters reached an extinction ratio of 2.55 10⁻⁶. The minimum extinction ratio is reached when the correct orientation of the second quarter-wave optical retarder is reached. In this position the second angle is 45 degrees (plus or minus 0.05 degrees). As mentioned above, the method to produce a matched pair of polarizing filters it is not required to measure the second angle. From Figure 3, it is clear that the minimum value of the extinction ratio is reached by rotating the second optical axis of the second quarter-wave optical retarder before securing the different components.

## Claims

1. A method to produce a matched pair of polarizing filters [132,134] comprising a first and a second polarizing filter, said first polarizing filter [132] comprising a first linear polarizer [124] and a first quarter-wave optical retarder [128] and said second polarizing filter [134] comprising a second linear polarizer [126] and a second quarter-wave optical retarder [130], said method comprising the steps of
- providing a beam of light [104] from a light source [102] along a propagation axis [101];
- providing a first [110], second [112], third [114] and fourth [116] rotation stage oriented perpendicular to said propagation axis [101] of the beam of light [104];
- mounting a first linear polarizer [124] having a first transmission axis in said first rotation stage [110] in a first position:
- mounting a second linear polarizer [126] having a second transmission axis in said fourth rotation stage [116];
- rotating said second linear polarizer [126] to obtain maximum extinction of said beam of light [104];
- inserting a first quarter-wave optical retarder [128] having a first optical axis in said second rotation stage [112];
- rotating said first quarter-wave optical retarder [128] to obtain maximum extinction of said beam of light [104];
- rotating said first optical axis of said first quarter-wave optical retarder [128] in a first direction over a first angle, said first angle being 45 degrees, preferably 45 degrees plus or minus 0.10 degrees;
- inserting a second quarter-wave optical retarder [130] having a second optical axis in said third rotation stage [114];
- rotating said second optical axis of said second quarter-wave optical retarder [130] in a second direction over a second angle to obtain maximum extinction of said beam of light [104], said second direction being opposite to said first direction of said rotation of said first optical axis of said first quarter-wave optical retarder [128] as viewed from said light source [102];
- securing said first linear polarizer [124] and said first quarter-wave optical retarder [128] together to form said first polarizing filter [132] and securing said second linear polarizer [126] and said second quarter-wave optical retarder [130] together to form said second polarizing filter [134].

2. A method according to claim 1, wherein said first polarizing filter [132] has a first handedness sense and said second polarizing filter [134] has a second handedness sense, with said first handedness sense and said second handedness sense being opposite as viewed from said light
source [102].

3. A method according to claim 1 or claim 2, wherein said matched pair of polarizing filters
[132, 134] has an extinction ratio lower than 10⁻⁵.

4. A method to analyze a sample [201] comprising birefringent particles suspended in a fluid, said method comprising the steps of
- producing a matched pair of polarizing filters [204, 206], comprising a first polarizing filter [204] and a second polarizing filter [206], according to the method of claim 1 or claim 2,
- providing a beam of light from a light source [202],
- introducing a sample [201] comprising birefringent particles suspended in a fluid between said first polarizing filter [204] and said second polarizing filter [206];
- passing said beam of light through said first polarizing filter [204], thereby creating a first beam of light;
- contacting said sample [201] with said first beam of light thereby creating a second beam of light;
- passing said second beam of light through said second polarizing filter [206], thereby creating a third beam of light;
- measuring the third beam of light by means of a detector [208].

5. A method according to claim 4, wherein said analyzing comprises determining the concentration of said birefringent particles suspended in said fluid.

6. A method according to claim 4 or claim 5, wherein said matched pair of polarizing filters is obtained by the method defined in claim 2.

7. A method according to any one of claims 4 to 6, wherein said birefringent particles comprise calcium carbonate, quartz, celestite, barite, kaolinite, chlorite, illite, vermiculite, orthoclase, plagioclase, montmorillonite, plastic or combinations thereof.

8. A method according to any one of claims 4 to 7, wherein said fluid comprises water or seawater.

9. An apparatus [200] for analyzing a sample [201] comprising birefringent particles suspended in a fluid. said apparatus [200] comprising a light source [202] for emitting a beam of light along a propagation axis [205], a matched pair of polarizing filters [204, 206] produced by the method defined in claim 1 or claim 2, said matched pair of polarizing filters [204, 206] comprising a first polarizing filter [204] and a second polarizing filter [206] and a detector [208], said light source [202], said matched pair of polarizing filters
[204, 206] and said detector I [208] being arranged such that said beam of light emitted from said light source
[202] subsequently can pass through said first polarizing filter [204], can impinge on the sample [201] to be analysed and can pass through said second polarizing filter [206] before being detected by said detector [208], said first polarizing filter [204] comprising a first linear polarizer and a first quarter-wave optical retarder and being configurable to polarize incident light into circularly polarized light of a first handedness sense viewed from the light source [202] and said second polarizing filter
[206] comprising a second linear polarizer and a second quarter-wave optical retarder and being configurable to polarize incident light into circularly polarized light of a second handedness sense, with said first handedness sense and said second handedness sense being opposite as viewed from said light source [202], said matched pair of polarizing filters [204, 206] having an extinction ratio lower than 10⁻⁵.

10. The apparatus [200] according to claim 9, wherein said matched pair of polarizing filters
[204, 206] is obtained by the method defined in claim 2.

11. The apparatus [200] according to claim 9 or 10, wherein said apparatus [200] is a transmissometer.

12. The apparatus [200] according to any one of claims 9 to 11, wherein said first linear polarizer has a first transmission axis, said first quarter-wave optical retarder has a first optical axis, said second linear polarizer has a second transmission axis and said second quarter-wave optical retarder has a second optical axis, with said first transmission axis, said second transmission axis, said first optical axis and said second optical axis each being oriented in a plane perpendicular to said propagation axis [205] of said beam of light.

13. The apparatus [200] according to any one of claims 12, wherein said first transmission axis and said second transmission axis are perpendicular to each other.

14. The apparatus [200] according to claim 12 or claim 13, wherein said first optical axis and said first transmission axis define a first angle and said second optical axis and said second transmission axis define a second angle, with said first angle and said second angle being 45 degrees plus or minus 0.10 degrees and with said first angle and said second angle having opposite signs as viewed from said light source [202].

15. The apparatus [200] according to any one of claims 10 to 14, further comprising one or more of the following components
- a beam splitter [212]; and/or
- one or more baffles [218]; and/or;
- one or more pressure windows [216, 220]; and/or
- one or more spectral filters [222]; and/or
- one or more lenses [210, 224]; and/or;
- one or more precision pinholes [209, 226].

## Patentansprüche

1. Verfahren zur Herstellung eines angepassten Polarisationsfilterpaars [132, 134], umfassend einen ersten und einen zweiten Polarisationsfilter, wobei der erste Polarisationsfilter [132] einen ersten linearen Polarisator [124] und einen ersten optischen Viertelwellenverzögerer [128] umfasst und der zweite Polarisationsfilter [134] einen zweiten linearen Polarisator [126] und einen zweiten optischen Viertelwellenverzögerer [130] umfasst, wobei das Verfahren die folgenden Schritte umfasst:
- Bereitstellen eines Lichtstrahls [104] von einer Lichtquelle [102] entlang einer Ausbreitungsachse [101];
- Bereitstellen einer ersten [110], zweiten [112], dritten [114] und vierten [116] Rotationsstufe, die senkrecht zu der Ausbreitungsachse [101] des Lichtstrahls [104] ausgerichtet sind;
- Montieren eines ersten linearen Polarisators [124], der eine erste Übertragungsachse in der ersten Rotationsstufe [110] aufweist, in einer ersten Position:
- Montieren eines zweiten linearen Polarisators [126], der eine zweite Übertragungsachse aufweist, in der vierten Rotationsstufe [116];
- Rotieren des zweiten linearen Polarisators [126], um eine maximale Extinktion des Lichtstrahls [104] zu erhalten;
- Einsetzen eines ersten optischen Viertelwellenverzögerers [128], der eine erste optische Achse aufweist, in die zweite Rotationsstufe [112];
- Rotieren des ersten optischen Viertelwellenverzögerers [128], um eine maximale Extinktion des Lichtstrahls [104] zu erhalten;
- Rotieren der ersten optischen Achse des ersten optischen Viertelwellenverzögerers [128] in einer ersten Richtung über einen ersten Winkel, wobei der erste Winkel 45 Grad, vorzugsweise 45 Grad plus oder minus 0,10 Grad, beträgt;
- Einsetzen eines zweiten optischen Viertelwellenverzögerers [130], der eine zweite optische Achse aufweist, in die dritte Rotationsstufe [114];
- Rotieren der zweiten optischen Achse des zweiten optischen Viertelwellenverzögerers [130] in einer zweiten Richtung über einen zweiten Winkel, um eine maximale Extinktion des Lichtstrahls [104] zu erhalten, wobei die zweite Richtung der ersten Richtung der Rotation der ersten optischen Achse des ersten optischen Viertelwellenverzögerers [128] von der Lichtquelle [102] aus gesehen entgegengesetzt ist;
- Sichern des ersten linearen Polarisators [124] und des ersten optischen Viertelwellenverzögerers [128] aneinander, um den ersten Polarisationsfilter [132] zu bilden, und Sichern des zweiten linearen Polarisators [126] und des zweiten optischen Viertelwellenverzögerers [130] aneinander, um den zweiten Polarisationsfilter [134] zu bilden.

2. Verfahren nach Anspruch 1, wobei der erste Polarisationsfilter [132] eine erste Händigkeitsausrichtung aufweist und der zweite Polarisationsfilter [134] eine zweite Händigkeitsausrichtung aufweist, wobei die erste Händigkeitsausrichtung und die zweite Händigkeitsausrichtung von der Lichtquelle [102] aus gesehen entgegengesetzt sind.

3. Verfahren nach Anspruch 1 oder Anspruch 2, wobei das angepasste Polarisationsfilterpaar [132, 134] ein Extinktionsverhältnis von weniger als 10⁻⁵ aufweist.

4. Verfahren zur Analyse einer Probe [201], umfassend doppelbrechende Partikel, die in einem Fluid suspendiert sind, wobei das Verfahren die folgenden Schritte umfasst:
- Herstellen eines angepassten Polarisationsfilterpaars [204, 206], das einen ersten Polarisationsfilter [204] und einen zweiten Polarisationsfilter [206] umfasst, gemäß dem Verfahren nach Anspruch 1 oder Anspruch 2,
- Bereitstellen eines Lichtstrahls von einer Lichtquelle [202],
- Einführen einer Probe [201], die doppelbrechende Partikel umfasst, die in einem Fluid zwischen dem ersten Polarisationsfilter [204] und dem zweiten Polarisationsfilter [206] suspendiert sind;
- Leiten des Lichtstrahls durch den ersten Polarisationsfilter [204], wodurch ein erster Lichtstrahl erzeugt wird;
- Inberührungbringen der Probe [201] mit dem ersten Lichtstrahl, wodurch ein zweiter Lichtstrahl erzeugt wird;
- Leiten des zweiten Lichtstrahls durch den zweiten Polarisationsfilter [206], wodurch ein dritter Lichtstrahl erzeugt wird;
- Messen des dritten Lichtstrahls mithilfe eines Detektors [208].

5. Verfahren nach Anspruch 4, wobei das Analysieren Bestimmen der Konzentration der doppelbrechenden Partikel, die in dem Fluid suspendiert sind, umfasst.

6. Verfahren nach Anspruch 4 oder Anspruch 5, wobei das angepasste Polarisationsfilterpaar durch das in Anspruch 2 definierte Verfahren erhalten wird.

7. Verfahren nach einem der Ansprüche 4 bis 6, wobei die doppelbrechenden Partikel Calciumcarbonat, Quarz, Celestit, Baryt, Kaolinit, Chlorit, Illit, Vermiculit, Orthoklas, Plagioklas, Montmorillonit, Kunststoff oder Kombinationen davon umfassen.

8. Verfahren nach einem der Ansprüche 4 bis 7, wobei das Fluid Wasser oder Meerwasser umfasst.

9. Vorrichtung [200] zur Analyse einer Probe [201], die doppelbrechende Partikel umfasst, die in einem Fluid suspendiert sind, wobei die Vorrichtung [200] eine Lichtquelle [202] zum Emittieren eines Lichtstrahls entlang einer Ausbreitungsachse [205], ein angepasstes Polarisationsfilterpaar [204, 206] umfasst, das durch das in Anspruch 1 oder Anspruch 2 definierte Verfahren hergestellt ist, wobei das angepasste Polarisationsfilterpaar [204, 206] einen ersten Polarisationsfilter [204] und einen zweiten Polarisationsfilter [206] und einen Detektor [208] umfasst, wobei die Lichtquelle [202], das abgestimmte Polarisationsfilterpaar [204, 206] und der Detektor [208] derart angeordnet sind, dass der von der Lichtquelle [202] emittierte Lichtstrahl anschließend durch den ersten Polarisationsfilter [204] geleitet werden kann, auf die zu analysierende Probe [201] auftreffen kann und durch den zweiten Polarisationsfilter [206] geleitet werden kann, bevor er durch den Detektor [208] detektiert wird, wobei der erste Polarisationsfilter [204] einen ersten linearen Polarisator und einen ersten optischen Viertelwellenverzögerer umfasst und konfigurierbar ist, um einfallendes Licht in zirkular polarisiertes Licht einer ersten Händigkeitsausrichtung, von der Lichtquelle [202] aus gesehen, zu polarisieren, und wobei der zweite Polarisationsfilter [206] einen zweiten linearen Polarisator und einen zweiten optischen Viertelwellenverzögerer umfasst und konfigurierbar ist, um einfallendes Licht in zirkular polarisiertes Licht einer zweiten Händigkeitsausrichtung zu polarisieren, wobei die erste Händigkeitsausrichtung und die zweite Händigkeitsausrichtung von der Lichtquelle aus gesehen entgegengesetzt sind [202], wobei das angepasste Polarisationsfilterpaar [204, 206] ein Extinktionsverhältnis von weniger als 10⁻⁵ aufweist.

10. Vorrichtung [200] nach Anspruch 9, wobei das angepasste Polarisationsfilterpaar [204, 206] durch das in Anspruch 2 definierte Verfahren erhalten wird.

11. Vorrichtung [200] nach Anspruch 9 oder 10, wobei die Vorrichtung [200] ein Transmissometer ist.

12. Vorrichtung [200] nach einem der Ansprüche 9 bis 11, wobei der erste lineare Polarisator eine erste Übertragungsachse aufweist, der erste optische Viertelwellenverzögerer eine erste optische Achse aufweist, der zweite lineare Polarisator eine zweite Übertragungsachse aufweist und der zweite optische Viertelwellenverzögerer eine zweite optische Achse aufweist, wobei die erste Übertragungsachse, die zweite Übertragungsachse, die erste optische Achse und die zweite optische Achse jeweils auf einer Ebene senkrecht zu der Ausbreitungsachse [205] des Lichtstrahls ausgerichtet sind.

13. Vorrichtung [200] nach einem der Ansprüche 12, wobei die erste Übertragungsachse und die zweite Übertragungsachse senkrecht zueinander sind.

14. Vorrichtung [200] nach Anspruch 12 oder Anspruch 13, wobei die erste optische Achse und die erste Übertragungsachse einen ersten Winkel definieren und die zweite optische Achse und die zweite Übertragungsachse einen zweiten Winkel definieren, wobei der erste Winkel und der zweite Winkel 45 Grad plus oder minus 0,10 Grad betragen und wobei der erste Winkel und der zweite Winkel von der Lichtquelle [202] aus gesehen entgegengesetzte Vorzeichen aufweisen.

15. Vorrichtung [200] nach einem der Ansprüche 10 bis 14, ferner umfassend eine oder mehrere der folgenden Komponenten:
- einen Strahlteiler [212]; und/oder
- eine oder mehrere Leitbleche [218]; und/oder;
- ein oder mehrere Druckfenster [216, 220]; und/oder
- einen oder mehrere Spektralfilter [222]; und/oder
- eine oder mehrere Linsen [210, 224]; und/oder
- eine oder mehrere Präzisionslochblenden[209, 226].

## Revendications

1. Procédé de production d'une paire de filtres polarisants appariée [132, 134] comprenant un premier et un second filtres polarisants, ledit premier filtre polarisant [132] comprenant un premier polariseur linéaire [124] et un premier retardateur optique quart d'onde [128] et ledit second filtre polarisant [134] comprenant un second polariseur linéaire [126] et un second retardateur optique quart d'onde [130], ledit procédé comprenant les étapes de
- fourniture d'un faisceau de lumière [104] à partir d'une source de lumière [102] le long d'un axe de propagation [101] ;
- prévision d'un premier [110], d'un deuxième [112], d'un troisième [114] et d'un quatrième [116] stade de rotation orienté perpendiculairement par rapport audit axe de propagation [101] du faisceau de lumière [104] ;
- montage d'un premier polariseur linéaire [124] présentant un premier axe de transmission dans ledit premier stade de rotation [110] dans une première position :
- montage d'un second polariseur linéaire [126] présentant un second axe de transmission dans ledit quatrième stade de rotation [116] ;
- rotation dudit second polariseur linéaire [126] pour obtenir une extinction maximale dudit faisceau de lumière [104] ;
- insertion d'un premier retardateur optique quart d'onde [128] présentant un premier axe optique dans ledit deuxième stade de rotation [112] ;
- rotation dudit premier retardateur optique quart d'onde [128] pour obtenir une extinction maximale dudit faisceau de lumière [104] ;
- rotation dudit premier axe optique dudit premier retardateur optique quart d'onde [128] dans une première direction sur un premier angle, ledit premier angle étant de 45 degrés, de préférence de 45 degrés plus ou moins 0,10 degrés ;
- insertion d'un second retardateur optique quart d'onde [130] présentant un second axe optique dans ledit troisième stade de rotation [114] ;
- rotation dudit second axe optique dudit second retardateur optique quart d'onde [130] dans une seconde direction sur un second angle pour obtenir une extinction maximale de la lumière dudit faisceau de lumière [104], ladite seconde direction étant opposée à ladite première direction de ladite rotation dudit premier axe optique dudit premier retardateur optique quart d'onde [128] tel que vu depuis ladite source de lumière [102] ;
- fixation, l'un à l'autre, dudit premier polariseur linéaire [124] et dudit premier retardateur optique quart d'onde [128] pour former ledit premier filtre polarisant [132] et fixation, l'un à l'autre, dudit second polariseur linéaire [126] et dudit second retardateur optique quart d'onde [130] pour former ledit second filtre polarisant [134].

2. Procédé selon la revendication 1, dans lequel ledit premier filtre polarisant [132] présente un premier sens de chiralité et ledit second filtre polarisant [134] présente un second sens de chiralité, ledit premier sens de chiralité et ledit second sens de chiralité étant opposés lorsqu'ils sont vus depuis ladite source de lumière [102].

3. Procédé selon la revendication 1 ou la revendication 2, dans lequel ladite paire de filtres polarisants appariée [132, 134] présente un taux d'extinction inférieur à 10⁻⁵.

4. Procédé d'analyse d'un échantillon [201] comprenant des particules biréfringentes en suspension dans un fluide, ledit procédé comprenant les étapes de
- production d'une paire de filtres polarisants [204, 206] appariée, comprenant un premier filtre polarisant [204] et un second filtre polarisant [206], selon le procédé de la revendication 1 ou de la revendication 2,
- fourniture d'un faisceau de lumière à partir d'une source de lumière [202],
- introduction d'un échantillon [201] comprenant des particules biréfringentes en suspension dans un fluide entre ledit premier filtre polarisant [204] et ledit second filtre polarisant [206] ;
- passage dudit faisceau de lumière à travers ledit premier filtre polarisant [204], créant ainsi un premier faisceau de lumière ;
- mise en contact dudit échantillon [201] avec ledit premier faisceau de lumière, créant ainsi un deuxième faisceau de lumière ;
- passage dudit deuxième faisceau de lumière à travers ledit second filtre polarisant [206], créant ainsi un troisième faisceau de lumière ;
- mesure du troisième faisceau de lumière au moyen d'un détecteur [208].

5. Procédé selon la revendication 4, dans lequel l'analyse comprend la détermination de la concentration desdites particules biréfringentes en suspension dans ledit fluide.

6. Procédé selon la revendication 4 ou la revendication 5, dans lequel ladite paire de filtres polarisants appariée est obtenue par le procédé défini dans la revendication 2.

7. Procédé selon l'une quelconque des revendications 4 à 6, dans lequel lesdites particules biréfringentes comprennent du carbonate de calcium, du quartz, de la célestine, de la barytine, de la kaolinite, de la chlorite, de l'illite, de la vermiculite, de l'orthoclase, du plagioclase, de la montmorillonite, du plastique ou des combinaisons de ceux-ci.

8. Procédé selon l'une quelconque des revendications 4 à 7, dans lequel ledit fluide comprend de l'eau ou de l'eau de mer.

9. Appareil [200] d'analyse d'un échantillon [201] comprenant des particules biréfringentes en suspension dans un fluide, ledit appareil [200] comprenant une source de lumière [202] pour émettre un faisceau de lumière le long d'un axe de propagation [205], une paire de filtres polarisants [204, 206] appariée produite par le procédé défini dans la revendication 1 ou la revendication 2, ladite paire de filtres polarisants [204, 206] appariée comprenant un premier filtre polarisant [204] et un second filtre polarisant [206] et un détecteur [208], ladite source de lumière [202], ladite paire de filtres polarisants [204, 206] appariée et ledit détecteur [208] étant agencé de sorte que ledit faisceau de lumière émis par ladite source de lumière [202] puisse ensuite passer à travers ledit premier filtre polarisant [204], puisse frapper l'échantillon [201] à analyser et puisse passer à travers ledit second filtre polarisant [206] avant d'être détecté par ledit détecteur [208], ledit premier filtre polarisant [204] comprenant un premier polariseur linéaire et un premier retardateur optique quart d'onde et pouvant être configuré pour polariser de la lumière incidente en lumière polarisée de manière circulaire d'un premier sens de chiralité vu de la source de lumière [202] et ledit second filtre polarisant [206] comprenant un second polariseur linéaire et un second retardateur optique quart d'onde et pouvant être configuré pour polariser de la lumière incidente en lumière polarisée circulairement d'un second sens de chiralité, ledit premier sens de chiralité et ledit second sens de chiralité étant opposés lorsqu'ils sont vus depuis ladite source de lumière [202], ladite paire de filtres polarisants appariée [204, 206] présentant un taux d'extinction inférieur à 10⁻⁵.

10. Appareil [200] selon la revendication 9, dans lequel ladite paire de filtres polarisants appariée [204, 206] est obtenue par le procédé défini dans la revendication 2.

11. Appareil [200] selon la revendication 9 ou 10, dans lequel ledit appareil [200] est un transmissomètre.

12. Appareil [200] selon l'une quelconque des revendications 9 à 11, dans lequel ledit premier polariseur linéaire présente un premier axe de transmission, ledit premier retardateur optique quart d'onde présente un premier axe optique, ledit second polariseur linéaire présente un second axe de transmission et ledit second retardateur optique quart d'onde présente un second axe optique, ledit premier axe de transmission, ledit second axe de transmission, ledit premier axe optique et ledit second axe optique étant chacun orientés dans un plan perpendiculaire audit axe de propagation [205] dudit faisceau de lumière.

13. Appareil [200] selon l'une quelconque des revendications 12, dans lequel ledit premier axe de transmission et ledit second axe de transmission sont perpendiculaires l'un à l'autre.

14. Appareil [200] selon la revendication 12 ou la revendication 13, dans lequel ledit premier axe optique et ledit premier axe de transmission définissent un premier angle et ledit second axe optique et ledit second axe de transmission définissent un second angle, ledit premier angle et ledit second angle étant de 45 degrés plus ou moins 0,10 degrés et ledit premier angle et ledit second angle présentant des signes opposés lorsqu'ils sont vus depuis ladite source de lumière [202].

15. Appareil [200] selon l'une quelconque des revendications 10 à 14, comprenant en outre un ou plusieurs des composants suivants
- un diviseur de faisceau [212] ; et/ou
- un ou plusieurs déflecteurs [218] ; et/ou ;
- une ou plusieurs fenêtres de pression [216, 220] ; et/ou
- un ou plusieurs filtres spectraux [222] ; et/ou
- une ou plusieurs lentilles [210, 224] ; et/ou ;
- un ou plusieurs trous d'épingle de précision [209, 226].
